# EUROPEAN PATENT SPECIFICATION

(11) **EP 0 253 629 B1**
(45) Date of publication and mention of the grant of the patent: **21.09.1994**
(21) Application number: 87306220.2
(22) Date of filing: 14.07.1987
(51) Int. Cl.: A61B 17/08, A61B 17/10, A61B 17/064

(54) **Staple**
Klammer
Agrafe

(30) Priority: 14.07.1986 US 885221
(43) Date of publication of application: 20.01.1988
(60) Divisional application: 92107161.9
(73) Proprietor: MINNESOTA MINING AND MANUFACTURING COMPANY, St. Paul, Minnesota 55133-3427 (US)
(72) Inventor: Froehlich, Harold E Minnesota Mining and, St. Paul Minnesota 55144-1000 (US); Foslien, Floyd L Minnesota Mining and, St. Paul Minnesota 55144-1000 (US)
(74) Representative: Baillie, Iain Cameron

(56) References cited:
- EP-A- 0 061 867
- EP-A- 0 085 930
- EP-A- 0 094 752
- EP-A- 0 229 453
- WO-A-79/00970
- WO-A-82/02486
- US-A- 4 403 693
- US-A- 4 470 532

## Description

### Technical Field

The present invention relates to shapes for open wire staples for suturing living tissue.

### Background Art

The art is replete with shapes of open wire staples for suturing living tissues, and rams and anvils of various shapes used to close such staples. U.S. Patent Nos. 3,873,016; 4,202,480; 4,256,251; 4,321,002; 4,477,007; 4,477,008; and 4,519,532 provide illustrative examples.

Such prior art open wire staples have included a staple having a straight central portion with straight pointed end portions projecting at right angles thereto (such as is illustrated in Figure 4 of U.S. Patent No. 4,202,480, which staple may be bent at two spaced locations along its central portion to form a generally rectangular closed staple (see Figure 6 of U.S. Patent No. 4,202,480) and is called a "box" staple herein. The side of the closed box staple opposite its end portions can subsequently be bent into a U-shape to retract the staple from the skin and underlying tissue as is illustrated in U.S. Patent 4,026,520. A further box staple is disclosed in EP-A-0229453 comprising a generally U-shaped central portion connected to an intermediate portion with an angle of 100-110° and the outer end of the intermediate portion is joined by an acute angle of 70-75° to outer legs which are parallel to one another and which terminate in points. The European application falls under Article 54(3) EPC. Such box staples have been widely used and have been well accepted by the medical community. This is at least partially so because of the currently preferred method for stapling skin. It is known that the inner surfaces of skin edge portions separated by an injury or an incision must make contact for proper healing to occur, and that if skin edge portions are inverted during stapling (i.e., positioned with their outer surfaces face to face) poor healing with the probability of scarring will result. Thus skin edge portions to be stapled together typically are everted (i.e., tented upwardly) with the inner surface of the skin edge portions pressed together, and the staple is applied over the everted skin edge portions to hold them together. When the box staple is so used, the parts of the skin edge portions pierced by the staple initially are positioned along its aligned end portions on the side of the closed box staple adjacent the skin portions being stapled. With time, tensions in the skin transverse to the opening being stapled closed will cause the parts of the skin portion pierced by the staple to move from around the adjacent end portions (which then become buried in tissue beneath the joined skin portions) to positions around the opposite side portions of the closed staple at which the skin edge portions lose some of their original eversion and are more in alignment. With the box staple, however, this movement of the skin does not occur immediately due to the restriction caused by the right angle corners between the end portions and side portions of the staple around which corners those parts of the skin portions must pass. Thus, the skin portions have a tendency to remain everted along the entire length of the opening being closed at least for the period of time required to complete stapling together of the skin edge portions, and a person who has completed the stapling can visually assure himself after the stapling of the skin edge portions is complete that the skin edge portions are still in a position which will afford proper healing. The box type staple, however, suffers from the disadvantage of requiring the staple to be bent sharply in two locations to close it in a manner which has required the use of metal rams and anvils and the application of substantial closing force; and causes some damage to the skin, particularly when the parts of the skin edge portions pierced by the staple move around the right angle bends in the staple as described above.

Another prior art staple for suturing living tissue described in U.S. Patent No. 4,321,002 comprises a generally U-shaped central portion having at least one arcuate part and curved outer portions terminating in sharp points. That staple can be closed by bending generally straight the arcuate part or parts so that the curved outer portions will smoothly enter and gather living tissue such as skin portions. During such closure the sharp points on the outer portions move to adjacent generally aligned positions to provide a generally D-shaped staple. As with the previously described box staple, the central portion of the closed D-shaped staple can subsequently be bent to retract the outer portions of the staple from the tissue.

The force needed to close such a D-shaped staple having only one arcuate part is substantially reduced for a similarly sized staple from the force required to close the box staple described above, so that the D-shaped staple can be closed with a ram and anvil made of polymeric material such as is described in U.S. Patents No's. 4,477,008 and 4,519,532. Also the curved outer portions can cause a reduced amount of damage to skin and tissue during use compared to the box staple. Such D-shaped staples, however, have not been received by the medical community with the enthusiasm one might expect presumably in part because the closed D-shaped staple will not hold joined skin portions in an everted position as long as a closed box staple. The stapled skin portions easily and quickly move along the arcuate portions of the D-shaped staple to a generally aligned position (which aligned position provides as good or better healing than the everted position) typically before the person stapling together the skin edge portions has completed the required stapling, so that that person can not observe all of the stapled skin edge portions in an everted position and thus be sure that some of the skin portions will not subsequently become inverted.

Also, even though the closing force of the "D" shaped staple having only one arcuate part is sufficiently low that rams and anvils used to close it can be made of polymeric material, significant wear can occur on such polymeric rams and anvils when they are used to close a large number of staples; and it has been found that staples can slip sideways along the surfaces of the polymeric rams and anvils contacting the staple (a phenomenon not observed between metal anvils, rams and staples) which can result in improperly shaped closed staples.

Another general problem that has existed in the design of rams and anvils for staplers has been to deal with the tendency for the central portion of the open staple to bend away from the anvil toward the ram as a result of forces by which the staple is bent to its closed position. One suggested approach for use with box staples has been to arch the center portion of the open staple toward the anvil so that it bends to a generally straight position as the staple is bent closed as is illustrated in U.S. Patent No. 4,256,251 (see Figures 12 and 19). Another approach being used is to shape the central portion of the ram so that it will straighten the bowed central portion of the staple by bending it against the anvil after the staple is essentially closed as is done in the "Precise"^{∼} stapler sold for several years by Minnesota Mining and Manufacturing Company, St. Paul, Minnesota. Yet another approach has been to provide a support to prevent such bending on the side of the central portion of the staple opposite the ram as is described in U.S. Patent No. 4,127,227.

However prevented, it is preferable that the central portion of the staple is not bowed significantly upwardly after the staple is closed, since if such a bow is present, pressure from the jaws of a staple remover of the type described in U.S. Patent No. 4,026,520 will cause crank-like forces tending to rotate the staple around its central portion as it is bent open, which rotation of the staple can cause the staple to be bent out of its plane which may damage the tissue from which the staple is being removed.

US-A-4,403,693 discloses an open wire staple comprising a generally U-shaped central portion having opposite ends and at least one arcuate part and outer portions terminating in sharp points. The outer portions each comprise a proximal part and a distal part with said sharp point being on the end of each said distal part opposite said proximal part, and the end of said proximal part opposite said distal part is disposed at substantially a right angle to and connected to one of said opposite ends of said central portion. The staple is closable by generally straightening the arcuate part of said central portion adjacent each of said outer portions and during such closure the distal parts move substantially into collinearity with one another. However in the closed form of the staple disclosed, although there is a central portion of the staple which is generally straight, portions of the staple adjacent the central portion are bowed downwardly which may give rise to the crank-like forces mentioned above unless the staple remover is accurately centered. Furthermore the closed staple is of the D-shape, and as mentioned above, this is disadvantageous in certain applications. Finally the sharp points of the closed staple are not overlapping each other and the gap between the points may result in reduced support at the location where the skin portions are joined.

### Disclosure of the Invention

The present invention provides a novel open wire staple for suturing living tissue that can be bent closed using a ram and anvil of polymeric material to form a rectangular or box shaped closed staple.

According to the present invention there is provided an open wire staple for suturing living tissue, said open staple comprising a generally U-shaped central portion having opposite ends and at least one arcuate part; and outer portions terminating in sharp points), said outer portions each comprising a proximal part and a distal part with said sharp point being on the end of said distal part opposite said proximal part, and the end of said proximal part opposite said distal part being disposed at substantially a right angle to and connected to one of said opposite ends of said central portion, said staple being closable by generally straightening the arcuate part of said central portion so that said outer portions can enter and gather living tissue, during such closure said distal parts moving substantially into collinearity with one another and the central portion of the closed staple subsequently being bendable to retract the outer portions of the staple from the living tissue, characterized in that: -
said proximal part and said distal part are substantially straight with said parts disposed at a right angle to each other;
such that said closed staple is of rectangular geometrically closed form with each said proximal part being disposed at substantially a right angle to said straightened arcuate part, and the sharp points overlapping each other.

The central portion of the closed staple can subsequently be bent to retract the outer portions of the staple from the living tissue in the manner illustrated in U.S. Patent No. 4,026,520.

Preferably for ease of closing the staple the U-shaped portion of the staple has only one arcuate part, however, two spaced arcuate parts may also be used which can provide more gather than a single arcuate part.

A stapler which can be used with the staple of this invention is claimed in our co-pending European Published Application No. 0497389.

### Brief Description of the Drawings

The present invention will be further described with reference to the accompanying drawings wherein like reference numerals refer to like parts in the several views, and wherein:
Figure 1 is a plan view of an open wire staple for suturing living tissue according to the present invention;
Figure 2 and 3 are plan views of the staple of Figure 1 closed in living tissue;
Figure 4 is a plan view of the staple of Figure 2 bent to an open position by a staple remover;
Figures 5 through 8 are fragmentary schematic views sequentially illustrating closing of the open wire staple of Figure 1 using a ram and anvil structure subject of our co-pending application.

### Detailed Description

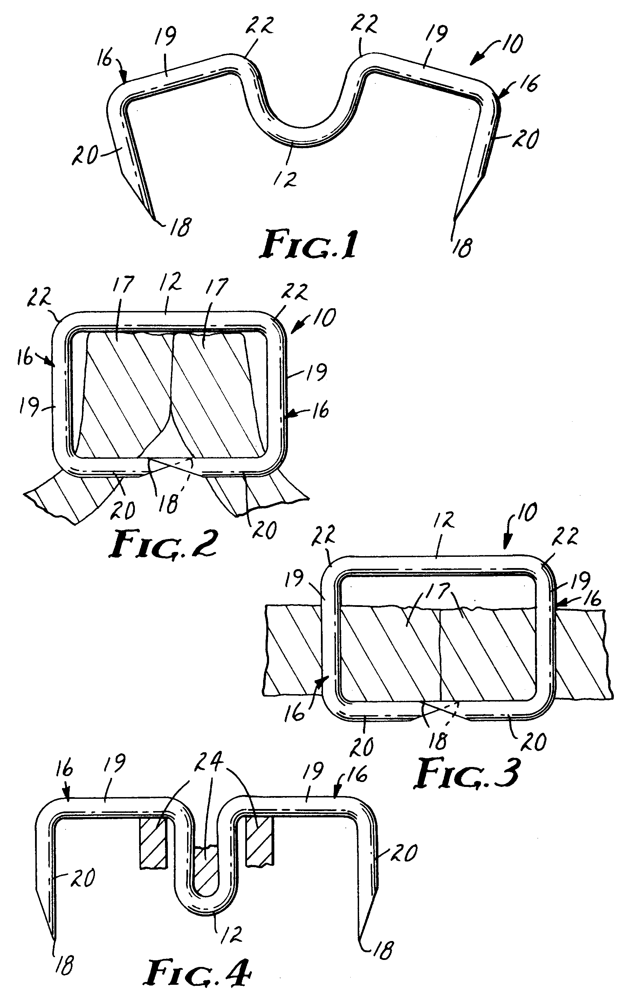

Referring now to Figure 1 of the drawing there is illustrated an unused open wire staple for suturing living tissue according to the present invention, generally designated by the reference numeral 10.

As is best seen in Figure 1, the open wire staple 10 comprises a generally U-shaped central portion 12 having one arcuate part; and outer portions 16 terminating in sharp points 18. The outer portions 16 each comprise a generally straight proximal part 19 and a generally straight distal part 20 with the parts 19 and 20 disposed at about a right angle to each other and with the sharp point 18 being on the end of each distal part 20 opposite the proximal part 19. The end of the proximal part 19 opposite the distal part 20 is disposed at about a right angle to and is connected at a first corner 22 to one end of the central portion 12. The staple is closable to the closed position shown in Figures 2 and 3 by bending generally straight the arcuate part of its central portion 12 so that the outer portions 16 can enter and gather living tissue such as adjacent skin edge portions 17. During such closure, the sharp points 18 on the distal parts 20 move to adjacent positions with the distal parts 20 generally aligned with each other to provide a generally rectangular or box shaped closed staple. Typically, the skin edge portions 17 are initially everted as shown in Figure 2 so that only the distal parts 20 pierce the skin edge portions 17. Subsequently, as described above in the Background Art portion of this specification, tension in the skin edge portions 17 causes them to move around the right angle corners between the distal parts 20 and proximal parts 19 to the generally aligned positions shown in Figure 3.

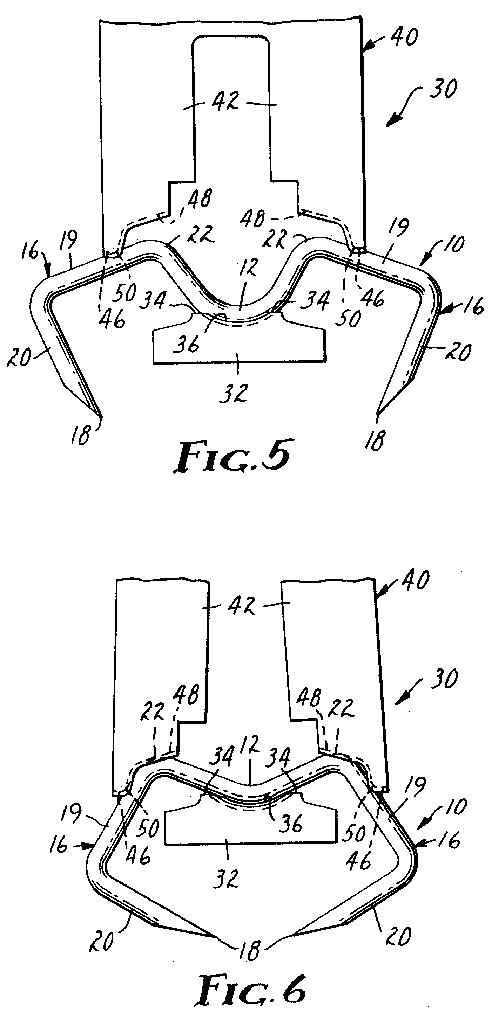

As shown in Figure 4, the central portion 12 of the closed staple can subsequently be bent to a U-shape by the jaws 24 of the type of staple remover described in U.S. Patent No. 4,026,520 to retract the outer portions 16 of the staple 10 from the skin portions 17.

The staple is preferably formed of a wire with a circular cross section (e.g. 0.57mm (0.0225 inch diameter)) and as can be seen in Figure 1, the central part of the central portion 12 of the unused open staple 10 is bent in a semicircular arc (e.g. 1.69mm (0.0666 inch)) radius to its outer surface). The outer parts of the central portion 12 diverge from parallel by about 30 degrees so that the proximal parts 19 of the staple 10 correspondingly are disposed to form an angle of about 150 degrees with each other. Alternatively the outer parts of the central portion 12 could be disposed between positions parallel with each other so that the proximal parts 19 are aligned, and portions diverging from parallel by about 60 degrees so that the proximal parts 19 form an angle of about 120 degrees with each other.

Referring now to Figures 5 through 8, there is schematically illustrated a stapler 30 for closing the staple 10, which stapler 30 may be in the form of the stapler illustrated in U.S. Patent No. 4,519,532

Generally, as illustrated, the stapler 30 comprises an anvil 32 having spaced support surfaces 34 and a recess 36 between the support surfaces 34 adapted to receive a central part of the generally U-shaped central portion 12 of the staple 10. Also, the stapler 30 comprises a ram 40 comprising an end portion of resiliently flexible material having two spaced and separated parts 42 each having a concave end surface adapted to engage the open staple 10. The end surfaces have spaced outer portions 46 and inner portions 48 recessed from the outer portions and extending generally transverse of the ram 40. The end surfaces are arcuate from their outer portions 46 to their inner portions 48 to form opposed arcuate surface portions 50 adapted to engage along the outer portions 16 of the open staple. The junctures between the radiused surface portions 50 and the inner surface portions 48 form sockets adapted to engage the first corners 22 of the staple 10 when the central portion 12 is partially straightened and the inner surface portions 48 are disposed to straighten the central portion 12 across the anvil 32 when the staple 10 to complete closure of the staple 10.

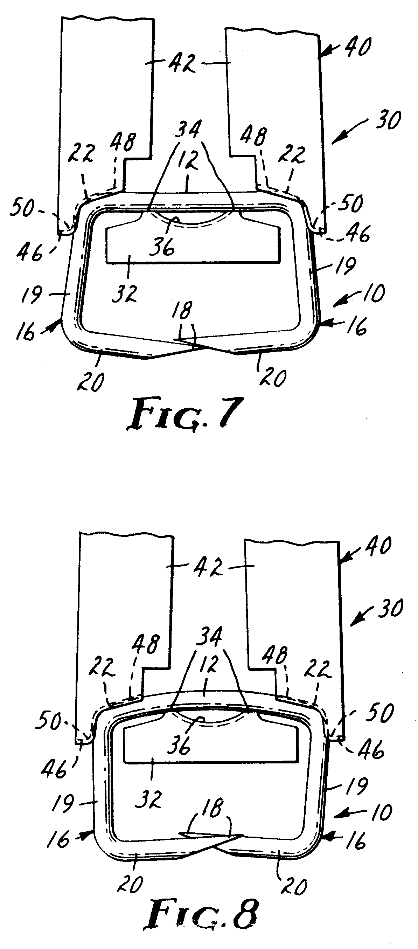

Also included in the stapler are means (not shown) for moving the ram 40 toward the anvil 32 to close the staple 10 around the anvil 32 with the central portion 12 of the staple 10 supported against the spaced support surfaces 34, which means may be in the form of the manually operable toggle joint linkage type drive described in U.S. Patent No. 4,519,532. Such movement of the ram 40 by such means will sequentially (1) press the radiused end surface portions 50 of the ram 40 against the proximal parts 19 of the outer portions 16 of the staple 10 (Figure 5) to resiliently spread the parts 42 of the ram 40 and apply forces both longitudinally and transversely of the ram 40 to generally straighten the central portion 12 of the staple (Figure 6); (2) to subsequently press the surfaces 50 and 48 forming the sockets against the first corners 22 of the staple 10 to generally straighten the central portion 12 (Figure 7); and (3) to then engage the inner surface portions 48 of the ram 40 with the central portion 12 of the staple to straighten the central portion 12 across the anvil 32 and complete closure of the staple 10.

The inner surface portions 48 of the ram are each disposed at a slight angle with respect to a line at a right angle to the longitudinal centerline of the ram 40 (e.g., 15 degrees) and are relieved opposite the support surfaces 34 which causes them to bend the central portion 12 of the staple into a slight arch around the support surfaces 34 of the anvil 32 (Figure 8). The elasticity in the staple wire will cause this arch to straighten when the pressure between the ram 40 and anvil 32 is removed, however, to provide a straight central portion 12 for the closed staple 10 as is shown in Figures 2 and 3.

Preferably the resiliency and geometry of the ram 40 are selected so that spreading of the parts 42 of the ram 40 during movement of the ram 40 between the positions shown in Figures 5 and 6 causes a resultant force to be applied to the proximal parts 19 of the staple 10 which is generally parallel to the axes of the generally straight end parts of the U-shaped central portion 12 and is spaced from the axis of those end parts on the sides of those end parts opposite the center of the U-shaped portion 12 a distance about equal the distance that would theoretically make such a force cause a long column the diameter of the staple 10 to buckle, so that the forces instead tend to straighten the central part of the central portion 12. While it is almost impossible to maintain precise force alignment of this type, it has been found that the closer such alignment can be maintained the less force is required to initially bend the staple 10 toward a closed position, which is particularly advantageous for a ram 40 driven by a toggle joint linkage type drive with which it is more difficult to develop force at the ram 40 during initial movement of the ram 40 than later in the movement of the ram 40.

The present Invention has now been described with referance to one embodiment thereof. It will be apparent to those skilled in the art that many changes can be made in the embodiment described without departing from the scope of the present invention. For example, the generally U-shaped central portion of the staple 10 can alternately be shaped like the central portion 60 of the staple 13 described in U.S. Patent No. 4,321,002 to have two spaced arcuate parts 61. This provides a staple that can provide more gather to pull together skin portions it engages, however, the staple cannot be closed by the type of ram 40 and anvil 32 illustrated above without some bending of the center of its central portions away from the anvil 32 toward the ram 40.

## Claims

1. An open wire staple (10) for suturing living tissue, said open staple comprising a generally U-shaped central portion (12) having opposite ends and at least one arcuate part; and outer portions (16) terminating in sharp points (18), said outer portions (16) each comprising a proximal part (19) and a distal part (20) with said sharp point (18) being on the end of said distal part (20) opposite said proximal part (19), and the end of said proximal part (19) opposite said distal part (20) being disposed at substantially a right angle to and connected to one of said opposite ends of said central portion (12), said staple being closable by generally straightening the arcuate part of said central portion (12) so that said outer portions can enter and gather living tissue, during such closure said distal parts (20) moving substantially into collinearity with one another and the central portion (12) of the closed staple subsequently being bendable to retract the outer portions (16) of the staple from the living tissue, characterized in that: -
said proximal part (19) and said distal part (20) are substantially straight with said parts (19, 20) disposed at a right angle to each other;
such that said closed staple is of rectangular geometrically closed form with each said proximal part (19) being disposed at substantially a right angle to said straightened arcuate part (12), and the sharp points (18) overlapping each other.

2. An open wire staple for suturing living tissue according to claim 1 wherein the U-shaped portion (12) of said staple has only one arcuate part.

3. An open wire staple for suturing living tissue according to claim 1 wherein the U-shaped portion (12) of said staple has two spaced arcuate parts.

## Patentansprüche

1. Offene Drahtklammer (10) zum Nähen von lebendem Gewebe, wobei die offene Klammer einen im allgemeinen U-förmigen mittleren Abschnitt (12) mit einander entgegengesetzten Enden und mindestens einen gebogenen Teil umfaßt; sowie äußere Abschnitte (16), die in scharfen Spitzen (18) enden, wobei die äußeren Abschnitte (16) jeweils einen proximalen Teil (19) und einen distalen Teil (20) umfassen, wo sich die scharfe Spitze (18) am Ende des distalen Teils (20) befindet, der dem proximalen Teil (19) entgegengesetzt ist, und das Ende des dem distalen Teil (20) entgegengesetzten proximalen Teils (19) im wesentlichen im rechten Winkel zu dem einen der einander entgegengesetzten Enden des mittleren Abschnitts (12) angeordnet und mit diesem verbunden ist, wobei die Klammer dadurch geschlossen werden kann, daß man den gebogenen Teil des mittleren Abschnitts (12) im allgemeinen streckt, so daß die äußeren Abschnitte in lebendes Gewebe eindringen und dieses zusammenhalten können, wobei die distalen Teile (20) sich während dieses Schließvorganges im wesentlichen kollinear zueinander bewegen, und der mittlere Abschnitt (12) der geschlossenen Klammer anschließend gebogen werden kann, um die äußeren Abschnitte (16) der Klammer aus dem lebenden Gewebe herauszuziehen, dadurch gekennzeichnet, daß:
der proximale Teil (19) und der distale Teil (20) im wesentlichen gerade sind, wobei diese Teile (19, 20) im rechten Winkel zueinander angeordnet sind;
so daß die geschlossene Klammer eine rechteckige, geometrisch geschlossene Form besitzt, wobei sich jeder proximale Teil (19) im wesentlichen im rechten Winkel zu dem gestreckten, gebogenen Teil (12) befindet, und die scharfen Spitzen (18) einander überlappen.

2. Offene Drahtklammer zum Nähen von lebendem Gewebe nach Anspruch 1, bei der der U-förmige Abschnitt (12) der Klammer nur einen gebogenen Teil besitzt.

3. Offene Drahtklammer zum Nähen von lebendem Gewebe nach Anspruch 1, bei der der U-förmige Abschnitt (12) der Klammer zwei voneinander beabstandete gebogene Teile aufweist.

## Revendications

1. Agrafe ouverte (10) en fil métallique, servant à suturer des tissus vivants, cette agrafe ouverte comprenant une partie centrale (12) pratiquement en U, comportant des extrémités opposées et au moins une zone courbe, et des parties extérieures (16) se terminant par des pointes aigues (18), ces parties extérieures (16) comprenant chacune une zone proximale (19) et une zone distale (20), la pointe aigue (18) étant située à l'extrémité de la zone distale (20) qui est située à l'opposé de la zone proximale (19) et l'extrémité de la zone proximale (19) située à l'opposé de la zone distale (20) étant disposée pratiquement à angle droit, en étant reliée à celle-ci, avec l'une des extrémités opposées de la partie centrale (12), l'agrafe étant agencée de façon à pouvoir être fermée en rendant pratiquement rectiligne la zone courbe de la partie centrale (12), d'une façon telle que les parties extérieures puissent pénétrer dans les tissus vivants et les rassembler au cours d'une telle fermeture, les parties distales (20) se déplaçant de façon à venir pratiquement dans l'alignement l'une de l'autre et la partie centrale (12) de l'agrafe fermée étant agencée de façon à pouvoir être ultérieurement pliée de façon à retirer hors des tissus vivants les parties extérieures (16) de l'agrafe, caractérisée en ce que la zone proximale (19) et la zone distale (20) sont pratiquement rectilignes, ces parties (19 et 20) étant disposées à angle droit l'une vis-à-vis de l'autre, de sorte que l'agrafe fermée a une forme géométrique fermée rectangulaire, chaque zone proximale (19) étant disposée pratiquement à angle droit avec la zone courbe (12) rendue rectiligne, tandis que les pointes aigues (18) se chevauchent l'une l'autre.

2. Agrafe ouverte en fil métallique servant à suturer des tissus vivants, selon la revendication 1, dans laquelle la partie en U (12) de l'agrafe ne comporte qu'une seule zone courbe.

3. Agrafe ouverte en fil métallique servant à suturer des tissus vivants, selon la revendication 1, dans laquelle la partie en U (12) de l'agrafe comporte deux zones courbes espacées.
